## Europäisches Patentamt

## European Patent Office

## Office européen des brevets

(19)

(11) Publication number: **0 051 451**
**B1**

(12) **EUROPEAN PATENT SPECIFICATION**

(45) Date of publication of patent specification: **13.08.86**

(51) Int. Cl.⁴: **A 61 F 2/24**

(21) Application number: **81305122.4**

(22) Date of filing: **29.10.81**

(54) **Low profile prosthetic xenograft heart valve.**

(30) Priority: **03.11.80 US 203804**

(43) Date of publication of application:
**12.05.82 Bulletin 82/19**

(45) Publication of the grant of the patent:
**13.08.86 Bulletin 86/33**

(84) Designated Contracting States:
**BE CH DE FR GB IT LI NL SE**

(56) References cited:
**DE-B-2 355 959**
**FR-A-2 451 189**
**GB-A-1 264 472**
**US-A-3 197 788**
**US-A-3 714 671**
**US-A-3 983 581**
**US-A-4 079 468**
**US-A-4 084 268**

(73) Proprietor: **SHILEY INCORPORATED**
**17600 Gilette**
**Irvine California (US)**

(72) Inventor: **Ionescu, Marian Ian**
**357 Street Lane**
**Leeds L517 6RU (GB)**
Inventor: **Lenker, Jay Alan**
**259 Fairview Street**
**Laguna Beach California (US)**
Inventor: **Rosenbluth, Robert Frank**
**24591 La Hermosa Avenue**
**Laguna Niguel California (US)**
Inventor: **Seiler, Louis, Jr.**
**17341 Forbes Lane**
**Huntington Beach California (US)**

(74) Representative: **Wood, David John et al**
**Pfizer Limited Ramsgate Road**
**Sandwich Kent CT13 9NJ (GB)**

-Courier Press, Leamington Spa, England.

## Description

The early development of prosthetic heart valves is well documented in papers given at symposia in 1960 and in 1968, published in "Prosthetic Heart Valves", Lyman A. Brewer III, Ed., Charles C. Thomas Publishing Co., Springfield, Illinois (1969), Second National Conference on Prosthetic Heart Valves; "Prosthetic Valves for Cardiac Surgery", K. Alvin Merendino, Ed., Charles C. Thomas Publishing Co., Springfield, Illinois (1961).

Lefrak and Starr recently surveyed the development of cardiac valve prostheses, E. A. Lefrak and A. Starr, "Cardiac Valve Prostheses", Appleton-Century-Krofts, New York, 1979 and the development of tissue heart valves has been comprehensively reviewed by Ioniscu, Marian I., "Tissue Heart Valves", Butterworths, Boston, 1979.

Great efforts have been expended in the development of tissue heart valve prostheses and in the development of supportive structures, or stents, for tissue valves. Representative of efforts to develop stents for tissue valves are the disclosures in the following United States patents: Patent 3,570,014, W D. Hancock, March 16, 1971; Patent 3,714,671, William Sterling Edwards et al, February 6, 1973; Patent 3,755,823, W. D. Hancock, September 4, 1973; Patent 3,983,581, William W. Angell, October 5, 1976; Patent 4,035,849, William W. Angell et al, July 19, 1977; Patent 4,079,468, Domingo Santo Liotta, March 21, 1978; Patent 4,084,268, Marian I. Ionescu et al., April 18, 1978; Patent 4,106,129, Alain F. Carpentier et al, August 15, 1978; Patent 4,172,295, Richard J. Batten, October 30, 1979 and Patent 4,192,020, Richard B. Davis et al, March 11, 1980. Other structures are also reported in the aforementioned treatises on heart valve developments.

A number of specific tissue valves are described in the following publications:

W. Sterling Edwards et al, "Mitral and Aortic Valve Replacement with Fascia Lata on a Frame", *Journal of Thoracic & Cardiovascular Surgery, Volume 58, No. 6,* December 1969, Pages 854—858; Ionescu, M. I. et al, "Heart Valve Replacement with Ionescu-Shiley Pericardial Xenograft", *Cardiology Digest,* June 1977, Page 45; Ionescu, M. I. et al, "Heart Valve Replacement with the Ionescu-Shiley Pericardial Xenograft", *The Journal of Thoracic and Cardiovascular Surgery, Volume 73,* Pages 31—42, 1977; Tandon, A. P. et al, "Long-Term Haemodynamic Evaluation of Aortic Pericardial Xenograft", *British Heart Journal, Volume 40,* Pages 602—607, 1978; Ionescu, M. I. et al, "Long-Term Clinical and Haemodynamic Evaluation of the Ionescu-Shiley Pericardial Xenograft Heart Valve", *Thoraxchirugie, Volume 26,* Pages 250—258, 1978; Ionescu, M. I. et al, "Long-Term Sequential Haemodynamic Evaluation of Right Ventricular Outflow Tract Reconstruction using a Valve Mechanism", *The Annals of Thoracic Surgery, Volume 27, No. 5,* May 1979; Ross, D. N., "Flexible Bioprosthetic Pericardial Heart Valve", *Thoracic & Cardiovascular Surgery, Volume 28,* Pages 150—152, 1980.

Particular techniques for preparing, handling and storing tissue valves are disclosed in U.S. Patents Nos. 3,966,401, Hancock et al, June 29, 1976, and 4,182,446, Penny, January 1980.

Some of the earliest heart valve prostheses were flexible two- or three-cusp valves in which the cusps were constructed of various types of fabric. Some of these flexible leaflet valves had good flow characteristics but most failed early. The leaflets tore, separated from the annulus, or become rigid due to fibrous tissue ingrowth. From about 1960 into the 1970's the trend was to mechanical valves. These ranged from the mechanically quite simple Starr-Edwards valve to the relatively sophisticated Bjork-Shiley valve and included a number of disc poppet valves. These mechanical valves generally dominated the market and are still very satisfactory for many applications. Tissue valves are however the preferred treatment where anticoagulation therapy is not tolerated by the patient.

In 1962, Donald Ross and Sir Brian Barratt-Boyes, working independently, were performing implantation of homograft tissue valves, some of which were free graft implants and some of which were mounted on supporting stents. Fully clothed covered rigid stents were used in some of these homograft valves.

In 1965, Drs. Binet and Carpentier, and their associates, implanted a specially prepared porcine aortic valve xenograft. These porcine valves were sterilized and treated, e.g. with formaldehyde, and were commonly attached to a metal stent. Experience showed that these valves were of short life, largely because formaldehyde was used as the cross-linking agent. Formaldehyde was found to create reversible cross links in the tissue, thereby allowing early breakdown of the tissue. Dr. Carpentier, in about 1968, established the concept of the bioprosthesis by substantially eliminating antigenicity of the tissue, principally by changing the preservative from formaldehyde to glutaraldehyde. Glutaraldehyde has been shown to create cross links of a more permanent nature than those created by formaldehyde.

A number of porcine bioprostheses and specially designed stents for supporting these prostheses resulted from the efforts of Warren Hancock et al. Generally, pig aortic valves are procured under clean conditions, placed in a cold, balanced electrolyte solution, excess tissue is trimmed and the xenografts are immersed in 0.2% glutaraldehyde. The leaflets are held in their normal valving position under pressure during the tanning process and each valve is sutured to a cloth covered stent by sutures. A number of designs and stent constructions for the Hancock type valve are exemplified in the aforementioned United States Patents Nos. 3,570,014 and 3,755,823. Stents for porcine valves were

developed by a number of other workers also, see, e.g., U.S. Patents Nos. 3,983,581; 4,035,849; 4,079,468 and 4,106,129.

Stents for supporting cusp valves of other tissue members, e.g. fascia lata and pericardium, have been developed by a number of workers, see, e.g., U.S. Patent 3,714,671, and Edwards et al, "Mitral and Aortic Valve Replacement with Fascia Lata on a Frame", supra. Much of the pioneering work in this area of valve development was done by Dr. Martin I. Ionescu and his associates, see, e.g., Bartek et al, "Frame-Mounted Tissue Heart Valves: Technique of Construction, *Thorax, Volume 29,* Pages 51—55, 1974; Ionescu et al, "Heart Valve Replacement with Ionescu-Shiley Pericardial Xenograft", *Cardiology Digest,* June 1977; Ionescu et al, "Heart Valve Replacement with Ionescu-Shiley Pericardial Xenograft", *The Journal of Thoracic and Cardiovascular Surgery, Volume 73,* Pages 31—42, 1977; Tandon et al, "Long-Term Haemodynamic Evaluation of Aortic Pericardial Xenograft, *British Heart Journal,* Volume 40, Pages 602—607, 1978; Ionescu et al, "Long-Term Clinical and Haemodynamic Evaluation of the Ionescu-Shiley Pericardial Xenograft Heart Valve", *Thoraxchirugie, Volume 26,* Pages 250—258, 1978; Ionescu, et al, "Long-Term Sequential Haemodynamic Evaluation of Right Ventricular Outflow Tract Reconstruction using a Valve Mechanism", *The Annals of Thoracic Surgery, 27,* 425—434, 1979; and Ionescu, Editor, "Tissue Heart Valves", Butterworths, 1979.

A number of improvements in the basic Ionescu tissue heart valve have been made. For example, a tissue heart valve has been developed which has a cloth-covered stent of special construction, in which the outflow annulus diameter of the valve is defined and limited by the positioning of a coaptation stitch on the inside of the supporting legs of the stent, as has been the practice since the early development of the Ionescu type tissue heart valve. Another improvement in the method for aligning the tissue of the cusps of the Ionescu type heart valve is described in U.S. Patent No. 4,172,295, which also discloses the coaptation stitch inside the stent legs. It has been the practice, in order to achieve a maximum flow orifice in valves of implantation diameters less than or equal to 23 mm, to splay the stent legs outwardly in an effort to achieve a full-flow orifice inside the coaptation stitches.

While these various modifications and improvements in the basic Ionescu valve over the years have solved some of the problems, there remains a number of problems which have not been solved. Among these problems are the limitations on the size of the flow annulus which can be obtained, with consequent increased pressure gradient across the valve. Perhaps the most important disadvantage of the three-cusp tissue valves of the prior art is the fact that in each cusp there are two points of stress due to three-dimensional flexure at about 4 o'clock and at about 8 o'clock in the lower arc of the cusp, i.e. in the lower right hand portion and the lower left hand portion of the closed cusp as viewed laterally, head-on, from the outside. A similar phenomena is created by creases which tend to concentrate stress in the areas of the crease.

The tissue valves of the prior art generally have relied upon a face-to-face meeting of the cusps in the closed position and, consequently, have required relatively high stent legs and, in general, the tissue valves of the prior art have trimmed the tissue around the outflow end so as to form a generally round regular cylindrical configuration. This approach has resulted in the concentration of stresses in fold areas or in the lower right and left hand portions of the cusps.

An additional potential problem is that stress is concentrated in the tissue in some areas where sharp bending of the tissue around the stent occurs. Some measures can be taken to relive this stress. The stress tends to be highest at points of maximum curvature such as around the tips of the stent legs because of the pinching of the tissue leaflets together inside and above the tip of the stent leg.

The problems described above are largely or entirely solved by the present invention.

The present invention comprises an improved tissue valve prosthesis of the Ionescu type. Thus the invention provides a prosthetic heart value (10) comprising: a stent (102) comprising an annular base (104) integrally formed with three upwardly extending legs (106, 108, 110), the space between the legs configured to form generally elliptically shaped scallops (112) having a depth U measured from the top of the legs (107, 109, 111) to the bottom of the scallop (112), the stent (102) circumferentially forming a substantially right cylinder having an interior diameter D with the legs (106, 108, 110) extending parallel to the axis of the cylinder, the lower edge of the base (104) forming scallops corresponding generally to the arc of the scallops (112) between the legs (106, 108, 110), the scallops of the lower edge of the base (104) and the scallops (112) between the legs vertically defining three generally elliptically shaped one-third portions of the base (104) between the respective upright legs (106, 108, 110); a fabric covering (120, 122, 124, 126) encompassing and following the configuration of the stent (102); an annular sewing ring (200) attached to the fabric covering (126) and extending outwardly from the base (104); a tissue valve element (20) formed of three tissue leaflets (30, 50, 70) joined to form a generally cylindrical three-cusp valving element (20), with said cusps (30, 50, 70) being in face-to-face contact in the closed position of the valve (10) along radial lines (133) from the center of the valve (10) to said legs (106, 108, 110); means (210) attaching the tissue valve element (20) around the fabric-covered stent (102); and a plurality of coaptation stitches (130, 132, 134) through ths tissue leaflets (30, 50, 70) adjacent the upper edges (40, 60, 80) thereof; characterised in that said coaptation stitches (130, 132, 134) are disposed directly above the tops of the fabric-covered stent legs (106, 108, 110) fixing the leaflets (30, 50, 70) to form three cusps, the tissue valve element (20) forms in the open position of the valve (10) a cylinder having an inside diameter at the top of the value (10) substantially equal to the inside diameter of the fabric-covered stent (102), and each of said leaflets (30, 50, 70) has a top edge (40, 60, 80) forming a raised truncated triangular form with a central plateau (46) and two sides (42, 44) diverging from each side of the plateau (46) to corners at their junctures with the sides (34, 36) of the leaflet (30), with said plateaus (46) extending upwardly midway between the legs (106, 108, 110) in the open position of the valve (10) and the upper edges (40, 60, 80) of the cusps (30, 50, 70) meeting in the closed position of the valve (10) with the plateaus (46) adjacent each other.

The prior art portion in the above is essentially derived from U.S. patent specification No. 4,084,268.

Our copending European divisional patent application No. 84101480.6 (0125393) is as filed directed to the following subject matter:—

A prosthetic heart valve comprising:

(a) a stent comprising:

(1) a ring with scallops formed therein defining an aperture with inflow and outflow ends; and

(2) a plurality of legs coupled to said ring extending upwardly toward said outflow end, said stent being dimensioned such that the quotient of the radius of carvature of mobile tissue measured at the point of greatest curvature divided by the thickness of said tissue is greater than or equal to five at all points of contact between said tissue and said stent;

(b) a plurality of tissue leaflets attached to said stent and to each other to form a valving element; and

(c) a plurality of coaptation means in the vicinity of the tips of said stent legs for causing joining of the edges of said tissue leaflets.

For convenience, in describing the valve of this invention, the outflow end of the valve is depicted at the top of the drawings and the valve is described in this configuration; thus, the upward or top portion of the valve would correspond to the outflow portion of the valve and the bottom would correspond to the inflow end of the valve.

In the prosthetic valve of this invention, the scallop depth U of the stent is between 50% and 65% of the stent inner diameter D. Optimally, the stent scallop depth U is from about 55% to about 62% of the diameter D. This U/D ratio is very important in providing a low profile tissue valve prosthesis which has optimum flexure and coaptation of the leaflets using tissue fixed in the unstressed state, and resultant maximum valve life.

The outflow end of the three cusps of the valving member are, in the closed position, adjacent in the center and in face-to-face contact with each other along radii defined by the legs. The upper or outflow ends of the valving element lie in substantially face-to-face contact between the interior faces of the cusps of the valving element by from 0 to 1 or 2 mm in the center and from 3 to 7 mm intermediate the center and the legs, along the radial contact lines.

A significant additional feature of the present invention resides in the preferred configuration of the stent legs wherein the ratio defined as the value of the radius of curvature of tissue around the inside top of said leg measured at the point of greatest curvature divided by the thickness of the tissue is approximately five or more. The width of the post top is approximately from 0.760 to 1.05 millimeters and the thickness of the cloth covered post top is approximately 2 millimeters. The tissue thickness in the preferred embodiment is approximately 0.3 millimeters. The tissue does not pinch together inside such narrow posts during closure at physiological pressure and an inside radius of curvature equal to or greater than 1.5 millimeters is obtained. The life of a valve using such a stent will be extended beyond that of a valve with a smaller radius of curvature.

The improved stent may be also designed so that the ratio of the radius of curvature of the tissue around the fabric-covered stent at a point between the upright stent legs divided by the thickness of the tissue is greater than or equal to five.

Additional strength to the legs is provided by inwardly projecting tabs which are, preferably, integrally formed at the tips of the legs, conforming to the width of the stent legs at their tips but narrower than the stent legs at the bottom of the tabs, said tabs extending both vertically down the leg for a predetermined distance and inwardly toward the center axis of the stent. Apertures are formed in the tabs through which the coaptation stitches pass to register the coaptation stitch directly above the stent leg.

The coaptation stitches can be at least in two embodiments. The first embodiment consists of two separate stitches, one passing through one aperture in the tab and defining a plane generally parallel to the long axis of the stent legs and tied off above the tips of the stent legs, and the other stitch passing through another aperture in the tabs and defining a plane which is generally perpendicular to the plane defined by the first stitch, said second stitch being tied off at the outside edge of the stent leg. The second embodiment of the coaptation stitch is a single stitch passing through a plurality of holes in the tabs which defines a plane which is parallel to the long axis of the stent leg. The second embodiment is the preferred embodiment and generally defines a figure eight pattern through a pair of holes in each tab.

The inward projection of the tabs toward, the center of the flow aperture of the valve is preferably also limited to prevent touching between the tissue leaflets and the bottom of the tabs. Generally this distance of inward projection is limited to forty thousandths of an inch (1.016 mm) in the naked stent, i.e., no more than forty thousandths inward projection of the bare plastic tab. The width of the tabs is also limited to aid in preventing the bottoms of the tabs from touching the tissue leaflets. In the preferred embodiment, the widths of the tabs remains constant throughout their length while the widths of the stent legs increases at points farther from the tips of the stent legs. In another embodiment, the widths of the tabs decrease at points further from the tips of the stent legs irrespective of the widths of the stent legs.

Figure 1 is a perspective view of a completed valve of this invention.

Figure 2 is an exploded view of the cloth covered stent and the valving element formed of three leaflets prior to attachment to the stent.

Figure 3 is a perspective view of the preferred stent configuration.

Figure 4 is a sectional view of the curvature of tissue around the covered stent leg taken along line 4—4 in Figure 2.

Figure 5 is a top view of the stent shown in perspective in Figure 3.

Figure 6 is a sectional view taken along section line 6—6 in Figure 5.

Figure 7 is a partial perspective view of the coaptation of the tissue leaflets with the stent leg and coaptation stitch.

Figure 8 is another partial perspective view showing the view of Figure 7 in a later stage of manufacture.

Figure 9 is a top plan view, viewed from the outflow end, of the completed valve in the fully open position.

Figure 10 is a sectional view of the curvature of tissue around the stent taken along section line 10—10 in Figure 2.

Figure 11 is a detailed view of an embodiment of the coaptation stitching.

Figure 12 is a detailed view of the preferred embodiment of the coaptation stitching.

Figure 13 is a detailed view of a typical tab and stent leg tip showing the relative widths of each.

Figure 14 is a detailed view of another embodiment of a typical tab and stent leg tip showing the relative widths of each.

Figure 15 is a top view and a sectional view, taken along the section line in the top view, of a prior art stent utilized in one of the improvements of the basic Ionescu valve, with the measurements thereof shown for purposes of comparison with the present invention.

Figure 16 is a flat plan view of the outline of one of the leaflets used in the present valving element, before the sewing of three such leaflets together to form a cylindrical valving member.

Figure 17 is a top plan view, viewed from the outflow end, of the completed valve in the fully closed position.

Figure 18 is a partial cross-section taken along line 18—18 in Figure 8 showing the closed cusps of the valve leaflets along the closure line.

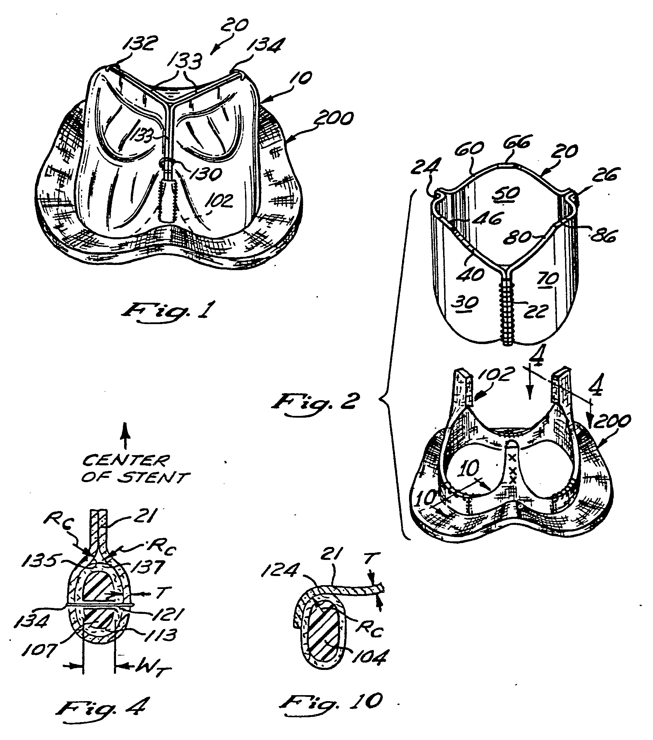

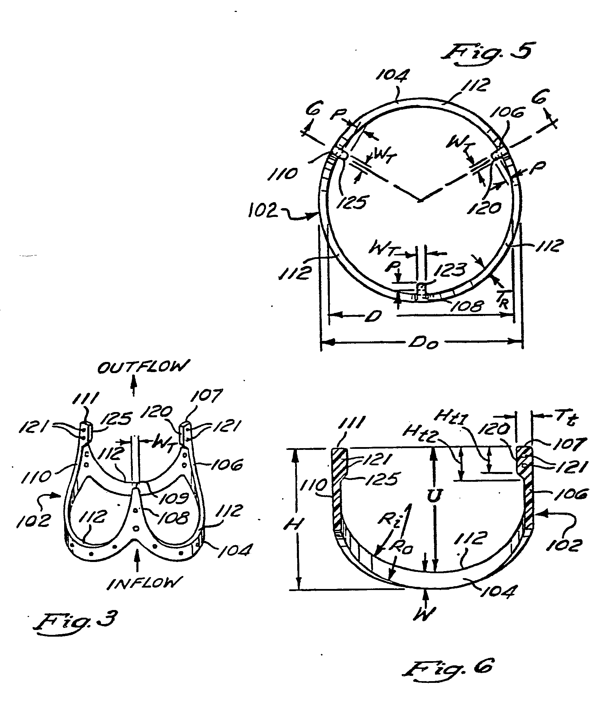

Figure 1 shows a low profile pericardial xenograft heart valve 10 which comprises a valving element 20, a stent assembly 102 and a suture or sewing ring assembly 200. The coaptation stitches 130, 132 and 134 cause coaptation or joining of the edges of the tissue leaflets of valving element 20 in the vicinity of the tips of the stent legs thereby forming radial coaptation lines 133 in Figure 1.

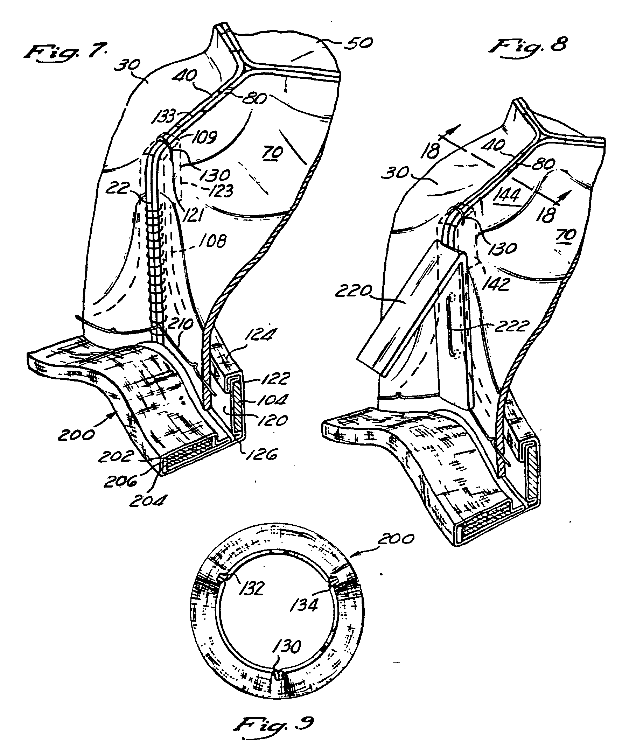

Figure 2 depicts an exploded view of the tissue valving element 20 prior to attachment of the stent 102, and generally shows the three stitch seams 22, 24 and 26 which join the three tissue leaflets 30, 50 and 70 into a right cylindrical valving element. This valving element is then sewn to the cloth-covered stent 102.

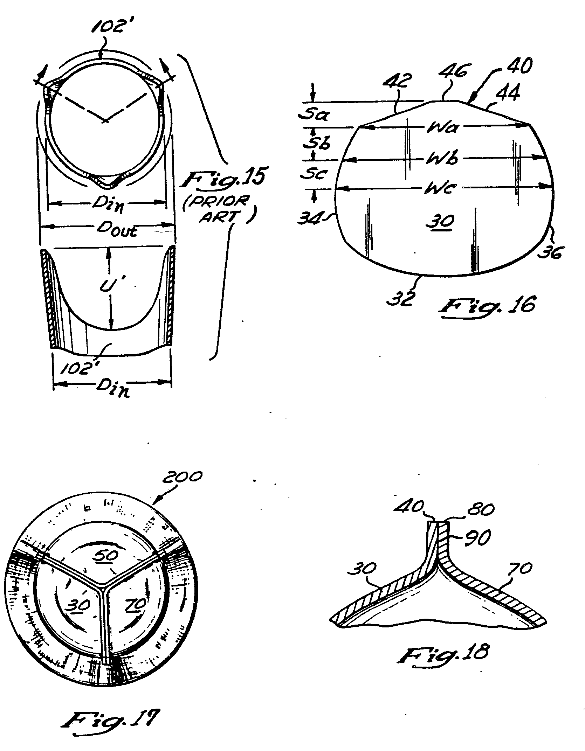

Figure 16 depicts one of the leaflets, leaflet 30, of the valving element 20, as exemplary of all of the leaflets 30, 50 and 70, all of which are substantially identical. The leaflet 30 is a generally flat layer or sheet of pericardium tissue, treated as will be described and discussed in more detail hereinafter, and includes a curved bottom 32 and curved sides 34 and 36 with a top, or outflow edge generally indicated at 40. The top 40, however, comprises three distinct portions. Edges 42 and 44 converge upwardly like symmetrical sides of a triangle to a central plateau 46 at the top and form obtuse corners with the sides 34 and 36, respectively, on the bottom. Each of the leaflets 50 and 70 are to be understood as including corresponding elements, including the top or outflow edges 60 and 80, as shown in Figure 2, for example, and plateaus 66 and 86 corresponding to plateau 46. The three leaflets 30, 50 and 70 are in all essential respects identical, although there will be some minor variation in the exact shape and size of these leaflets because they are made from naturally occurring tissue and considerable manual dexterity and skill is required in production. Minor variations, so long as the function is not impaired, are readily tolerated in the present valve construction.

The shape and interaction of the leaflets 30, 50 and 70 is very important to the proper functioning of the valve, although precise dimensions are not critical because minor deviations and dimensions can be compensated for in the final joinder of the leaflets into the valving element and in fitting the valving element over the stent. One aspect of the configuration of the leaflets is highly preferred for the optimum functioning of the present invention, although the invention will function in an improved manner over the prior art even if deviations are permitted. This preferred aspect of the configuration is the truncated triangular top edge converging to the plateau 46 in Figure 16 and to plateaus 66 and 86 in the leaflets 50 and 70. The base of the triangle is, of course, an imaginary line joining the lower corners of the top edge. Plateau 46 is centrally located between the juncture of the outflow edge portion 42 with side 34 and outflow edge portion 44 with side 36. The best definition of the shape of this obtuse truncated triangle defined by the plateau 46 and the juncture of the top edges 42 and 44 with the sides of the leaflet, is that this truncated triangle is so configured and dimensioned that when the three leaflets are sewn together at their respective edges to form a cylinder, and fitted over a stent, with a coaptation stitch positioned directly over the end of the stent when the valve is closed, the plateau 46 of the leaflet 30 touches, or substantially touches, the corresponding plateaus 66 and 86 of the leaflets 50 and 70, with no more than about 1 mm of face-to-face contact, in the center of the flow path of the valve with substantial face-to-face contact, i.e. from about 2 mm to about 6 or 7 mm, between the interior surfaces of the edges intermediate the center and the outer diameter of the valve. Exactitude is not perfectly required, but it is required that the plateaus 46, 66 and 86 be in touching or substantially in touching relationship when the valve is closed, and that there be substantial surface-to-surface contact along the central portion of the radial coaptation lines of cusp contact. This relationship is shown in Figure 18 which depicts the valve of Figure 1 cut perpendicular the radius defined by the cusp coaptation line 133, the area of contact being shown at 90. The maximum face-to-face contact is about halfway between the center and the legs and would be at least 2 or 3 mm but not more than 9 or 10 mm, optimally from 3 to 7 mm depending on valve size.

It is neither necessary nor possible to give exact shape and dimensional definitions to the leaflets exemplified by leaflet 30, but the configuration may be described, realizing that the truly critical relationship is the interrelationship of the three obtuse truncated triangular portions. The maximum width of the leaflet lies about mid-height thereof. The height of the leaflet is, of course, of no criticality whatever, and so this is merely a general relationship. Thus, the sum of Sa, Sb and Sc (see Figure 16) is approximately equal to one-half of the total vertical height of the leaflet, Sa representing the mean altitude of the obtuse truncated triangle formed by plateau 46, converging edge portions 42 and 44 and the base defined by the junctures of top edge 40 with side 34 and side 36, respectively, Sb plus Sa being equal to about 35% plus or minus 3 to 5% of the total vertical height, and the sum of Sa, Sb and Sc being about 50% plus or minus around 10% of the total vertical height. The width of the leaflet, Wa, measured Sa down from the plateau 46 is about 85% plus or minus 10% of the maximum width, Wc, of the leaflet, Sa being around 12 to 17% of the total vertical height. The width Wb measured at Sa plus Sb from the plateau 46 is about 95% plus or minus about 5% of the maximum width. The exact width and height ratios depend generally upon the overall size of the valve and will usually fall within the ranges indicated, although the first definition by function is the best and most meaningful description presently comprehended. In a specific embodiment, the valving member for the size 23 valve is a section of pericardium 0.012 inch thick, with a maximum height of 21 millimeters and a maximum width Wc of 26.5 millimeters at about 52% of total height. The width Wa of the obtuse triangle is 22.5 millimeters measured at Sa down about 14% of the total height from the top, the intermediate width Wb being 25.5 millimeters at Sb, 35% from the top. Again, this is merely one example of one size of a valve and the dimensions are not the critical factor; it is the interrelationship of the top edges of the leaflet that is critical.

Glutaraldehyde has been used effectively to stabilize connective tissue for clinical heart valve substitutes for several years. The tissue leaflets of the present invention are cut from pericardium tissue, although other tissues may be used. In the preferred embodiment of the present invention, pericardium treated with .5% glutaraldehyde at pH 7.4 without fixing the tissue in a prestressed condition is preferred; however, it is to be understood that the invention disclosed and claimed here relates to the configuration of the valving leaflets and element and the supporting stent, and any suitably preserved tissue may be utilized in the present invention.

The stent assembly refers generally to the entire stent assembly which includes a biologically compatible metal or plastic stent 102. The stent 102 defines the configuration of the stent assembly. The stent 102 may be considered as three one-third portions of a stent integrally formed of one piece of material although, of course, the method of formation or the number of pieces is of no consequence provided the completed stent is as described herein. The stent 102 comprises an annular base or ring 104 which extends around and defines the flow orifice of the valve. Coupled to the ring or integrally formed therewith are a plurality of stent legs extending upwardly a distance H toward the outflow end of the valve from the lowermost portion of the base. There are three substantially identical legs 106, 108 and 110, each separated from its neighboring legs by scallops 112 as best shown in Figure 3. The bottom or inflow edge of the stent 102 is also scalloped to conform generally to the arc of the scallops between the legs. These bottom scallops generally follow the configuration of the scallops 112 of the outflow edge so as to generally form parallel edges defining ring or base 104. The scallops of the lower or inflow edge of the base and the scallops of the outflow edge between the legs vertically define three generally elliptical shaped one-third portions of the base between the centerlines of the respective upright legs which together circumferentially form a right cylinder of constant diameter having an inside diameter D with the legs extending parallel to the axis of the cylinder as shown in Figure 5.

The stent assembly also includes a fabric covering which encloses the stent 102. It has been long recognised that there are structural and biological advantages to the use of fully cloth-covered stents for supporting tissue valves. This concept predates the present invention, and it is sufficient here to describe the stent assembly as including a cloth covering which encloses or substantially encloses and conforms to the stent.

Figure 7 is a partial cross-section depicting a fabric 120 enclosing the outside of the stent, a fabric 122 which encloses the inside of the stent, with a suitable seam area 124 joining the fabrics along the top or outflow edge of the stent. A fabric 126 is joined along the lower edge of the stent and extends outwardly forming part of and attaching a sewing or suture ring generally indicated at 200 which may be of any of the forms used in the prior art.

Generally, such suture rings comprise a plurality of layers of fabric and padding, 202, 204 and 206, enclosed in layer 126, soft enough to permit the suturing needle to be readily inserted through it and yet rigid and strong enough to provide firm mounting of the prosthesis in the heart valve area. The suture ring 200 of this invention differs from the prior art suture rings only in that it curves and conforms to the scalloped contour of the valve ring or base defined by the portion 104 of the stent 102.

The tissue leaflets, after being sewn to form a cylinder as shown in Figure 2, may be sewn to the stent assembly in any conventional manner, as, for example, by running stitches shown in 210 in Figure 7.

Figure 7 depicts the valve in a partially completed configuration with the tissue leaflets 30 and 70 joined by seam 22, the upper edges 80 and 40 substantially touching or just touching, without a large or significant surface-to-surface contact of the two leaflets. Coaptation stitch 130 is disposed directly over the tip 109 of the stent leg 108 and passes through a hole 121 in the tab 123 to form the radial coaptation line 133.

Figure 8 shows another stage in the construction of the valve shown in Figure 7 by the addition of the pledget and cover 220. This cover is sewn by stitches 222 to the stent leg through the tissue, or it can be connected in any other convenient manner.

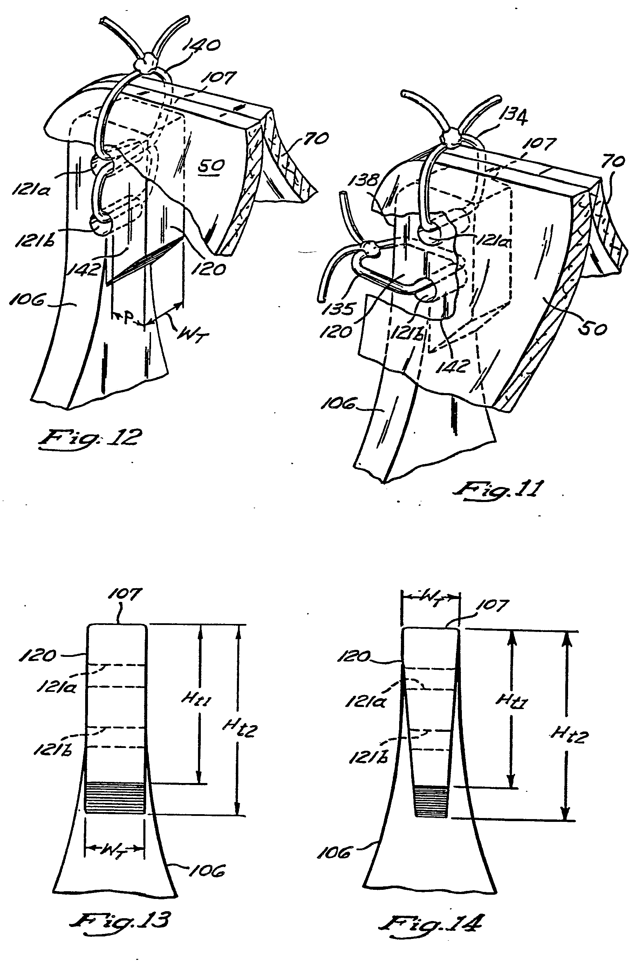

Coaptation of the tissue leaflets is caused by the action of coaptation stitches 134, 130 and 132 shown in Figure 1. The present invention departs from the prior art in a very important and significant manner in the way in which the coaptation of the edges 40 and 60, the edges 40 and 80, as best shown in Figure 18, and the edges 60 and 80 abut in touching relationship. This coaptation is defined generally by the placement of the coaptation stitches 134, 130 and 132 directly above the respective stent legs 106, 108 and 110, the placement of the coaptation stitch 130 being depicted as exemplary in Figures 7 and 8. In prior art valves such as the one disclosed in U.S. Patent 4,084,268, the coaptation stitch had been placed inside the circumference of the circle defined by the tips of the stent legs. Placement of the coaptation stitch directly above the tips of the stent legs tends to allow the orifice diameter of the fully open valve to equal the inside diameter of the covered stent. Figure 9 illustrates a tissue valve in the fully open position.

Another extremely significant departure from the prior art is the relative height H of the stent 102, the depth U of the scallops between the legs of the stent 102, and the inside diameter D of the stent.

In the prior art it was considered necessary, or at least very important, that in smaller valve sizes, e.g. 23 mm or less, the legs be splayed outwardly from the base. Thus, referring to Figure 15, in the prior art stent 102', the input diameter $D_{in}$ was smaller than the outflow diameter $D_{out}$, $D_{out}$ being the diameter of the circle in which the legs at the outflow end of the valve lie. The coaptation stitches of the prior art were formed inside the legs, and the diameter of the circle on which these coaptation stitches were made to lie was made, or attempted to be made, approximately equal to $D_{in}$. Thus, the stent, viewed circumferentially, e.g. from the end, was not a right cylinder, but was generally frustoconical because of the splaying. Sometimes, of course, the splaying of the legs was accomplished by bending the legs out from another wide cylindrical base, but the result was substantially the same as a frustoconical imaginary figure derived from the diameter of the inflow and the outflow ends of the valve. .

In contrast to the prior art, the stent 102 of the present invention is, viewed circumferentially, a right cylinder, with the axis of the cylinder lying in the center of the flow path and the legs extending from the inflow end toward the outflow end (the top as viewed in the figures) of the valve parallel to the axis of the right cylinder. Thus, $D_{in}$ becomes equal to $D_{out}$.

Also of great significance is the ratio U/D, D being equal, of course, to the inner diameter of the stent. As compared with what is regarded as the closest and most pertinent prior art, the Ionescu type valve described by Ionescu et al in U.S. Patent 4,084,268, various features of which are also described in U.S. Patent No. 4,172,295 to Batten, the scallop depth U measured from the scallop bottom on the outflow edge of the stent base 104 to the upper or outflow end of the stent legs (see Figure 6), is very much less, for a given valve diameter, than the corresponding distance U' in the prior art stent depicted in Figure 15. In particular, the ratio of U/D in the present valve is between about .50 and about .65, and optimally from about .55 to .62.

It is important, of course, to obtain and maintain as low a profile valve as can be made to operate; but that alone is not the only significance of the aforesaid ratio of U/D. This result, long sought for but heretofore unattainable, is obtained by reason of the unique combination of elements, configurations, relationships, and dimensional ratios, which, acting together in a unique way, make it possible to provide a heart valve prosthesis, in which the valving element is a generally cylindrical tissue element, which has a profile of less than two-thirds the profile of prior art valves, which closes more rapidly than prior art valves of related construction, and in which the stresses in the cusps of related prior art valves have been wholly or substantially avoided. This new result comes about by reason of the interaction and cooperative action and function of the U/D ratio of the stent, the positioning of the coaptation stitch above the end of the stent leg, and the unique configuration of the cusp leaflets of the valving element.

As will be seen in Figure 9, when the valve is in the fully open position, the flow path is substantially a right cylinder through the valve, with the coaptation stitches being placed directly above the legs. This has two functions. The first is of significance but, comparatively, of lesser significance than the other. The first result of this placement of the coaptation stitches is that a larger flow orifice is obtained without the necessity for splaying the legs of the valve. More importantly, the stresses of the prior art tissue valves at four o'clock and at eight o'clock, i.e. at the lower right and the lower left-hand portions of the cusp, when viewing the cusp straight on laterally, have been avoided without fluttering, rolling and floating of the edges of the tissue at the outflow end of the valve. This is a new and extremely desirable result which follows from the combination of configurations described. This result is accomplished by reason of the unique configuration of the leaflets in which the valve element comprises three tissue leaflets joined to form a generally cylindrical valve element having three points which extend centrally of each of the cusps respectively toward the outflow end of the valve and centrally between the ends of the legs, the valve element forming in the open position a cylinder having three points on the outflow end thereof and, in the closed position, forming three cusps which arc inwardly between the legs with the outflow end of the leaflets touching with the three points adjacent each other in the center of the outflow end of the valve. It will be apparent from a consideration of the structure of the leaflets that, while in the preferred embodiment they are formed of three separate pieces, they may very well be formed of a single integral piece of tissue with appropriate cutting and stitching such that the end result, the valving element, has the proper configuration. Thus, while it is convenient to start with three pieces of tissue, the same invention may be practiced with only one piece in which the three leaflets are integrally joined.

The shortening of the implant depth, to less than about two-thirds of the prior art stent heights of corresponding valves, and the adoption of the coaptation stitch directly above the ends of the legs, permit the use of the above described valving element while providing a substantial area of face-to-face overlapping contact along the radial contact lines of the cusps and obviating the tendency of the outflow ends of the leaflets to roll, flutter, and otherwise to delay in closing or to twist and deform by minimizing the coaptation, at the center of the valve, of the plateaus 46, 66 and 86.

A preferred stent design for tissue heart valves is depicted in detail in Figures 3, 5 and 6. Figure 3 shows the improved stent in perspective. Figure 5 shows a top view of the stent, and Figure 6 is a sectional view of the stent taken along section line 6—6 in Figure 5. It is advantageous to use this preferred stent design in conjunction with the valving element 20 described above. Such a combination is a preferred method of valve construction in the present invention. However, the preferred stent design may be employed with other valving elements, and, on the other hand, the valving element 20 may be used with other stents.

The width $W_t$ of the stent legs 106, 108 and 110 at their tips 107, 109 and 111, as viewed in Figure 5, is substantially less than the width of the tips of prior art stent legs. As shown in Figure 4, which is a detailed sectional view of the tip 107 of stent leg 106, the width $W_t$ is equal to the diameter of the half circle defining the tip if the tip is rounded. In other embodiments where the tip is not rounded, $W_t$ is measured between the points near the tip where the edges of the stent leg first start to curve in toward the center line of the stent leg.

In the preferred embodiment of the stent design, the stent legs are rounded at their tips. The width of the stent legs at the tips thereof has been reduced from approximately 2.032 millimeters in prior stents to a substantially narrower range of widths from about 0.76 mm to about 1.14 mm. This reduced width of the stent legs at their tips has the effect of increasing the radius of curvature of the tissue inside the stent leg and tip. This curvature is caused by the action of the coaptation stitches 134, 130 and 132 in Figure 1 and by closure of the valve which causes collapse of the cusps 30, 50 and 70 toward the center of the valve under restriction of the coaptation stitches. That is, the tissue leaflets 30, 50 and 70 curve less sharply together at the tips 107, 109 and 111 of the stent legs when the tips of the legs are narrower. This increased radius of curvature translates into reduced stress in the tissue of the cusps and a longer service life for the valve.

However, this reduced width of the tips of the stent legs 106, 108 and 110 in Figure 3 leaves less material in the stent legs to carry the structural loading of the closed leaflets caused by the impulse pressure in the flow of blood caused by intermittent pumping action of the heart. To compensate for the reduced width in the leg tips, tabs 120, 123 and 125 (see Figures 3 and 5) are added at the outflow end or tip of each of the stent legs to add structural strength to the legs. These tabs preferably project inwardly toward the center of the stent and are integrally formed with the stent legs and preferably conform to the width $W_T$ at the tips of the stent legs as seen in Figure 5. The tabs extend both vertically down the stent leg for a distance $H_{t1}$, Figure 6, and toward the center of the aperture defined by the stent ring for a distance $T_t$.

The exact dimensions of the tabs, $W_t$, $H_{t1}$, $H_{t2}$ and $T_t$ in Figures 5 and 6, are, within the parameters of this aspect of the invention, matters of design choice. The strength of material used in constructing the stent will affect the choice. Typical dimensions in the preferred embodiment of the stent design are given in Table I below.

The tabs 120, 123 and 125 have apertures 121 formed therein through which the coaptation stitches pass. It is preferred that the coaptation stitches be passed through the holes 121 in the tabs since this registers the position of the coaptation stitch at a positive, repeatable location. The diameter of the holes 121 in the preferred embodiment is 0.813 mm, and the dimension $T_t$ in Figure 6 is chosen to give sufficient strength. The use of holes 121 in the tabs located as described above insures good repeatability of manufacture because different assembly workers cannot change the orifice diameter or induce stresses in the tissue by inadvertent mislocation of the coaptation stitch too far toward or away from the center of the aperture.

The tabs 120, 123 and 125 are included in the preferred embodiment because they have been found to be highly desirable and necessary, in many instances, for sufficient strength; however, the tabs are not always necessary. With refined manufacturing techniques and adaptation of stronger materials, the tabs are expected to be eliminated or reduced in size. The fundamental concept disclosed herein is extension of the service life of the valve by reducing stress levels in the tissue by, among other things, utilizing narrower, rounded stent leg tips 107, 109 and 111 as shown in Figures 3 and 4. Ideally, a substantially zero tip width would be desirable, but structurally this is impossible at present. The width of the stent leg at its tip is made more narrow than heretofore known; however, the stated width of the tips of the stent legs in the preferred embodiment should not be understood as limiting the invention in any way.

A general guideline for the narrowness of the stent leg tip is that the tip should be sufficiently narrow so that the quotient of the radius of curvature of tissue together inside the stent leg tip divided by the thickness of the tissue is greater than or equal to five. The radius of curvature $R_c$ of the tissue inside a typical stent leg tip is illustrated in Figure 4. This curvature is caused by the coaptation of the leaflets under pressure and tends to concentrate shear stresses in the tissue at points 135 and 137 where curvature is greatest. In the preferred embodiment, this ratio should be a minimum of approxmately five. That is, if the radius of curvature of the tissue around the tip of the stent leg is less than approximately five times the thickness T of the tissue, then the tips of the stent legs are too wide. This general guideline has been shown to increase the service life of the valve; but the foregoing statement should not be understood as limiting the invention to a ratio of five.

It is critical that the stent dimensions be selected such that touching between the tissue and the stent is substantially eliminated or minimized, and the radius of curvature of the tissue around the stent is not smaller than a predetermined value. That is, touching between the tissue and the stent is substantially eliminated or minimized, and the radius of curvature of the tissue around the stent measured at the point of greatest curvature divided by the thickness of the tissue should be greater than or equal to five. This curvature at two of the various places on the stent where it occurs is illustrated in Figures 4 and 10. Figure 4 is a sectional view taken along section line 4—4 in Figure 2 looking down on the top of the stent leg. The center of the stent is toward the top of Figure 4. The radius lines $R_c$ illustrate the radius of curvature of the tissue 21 together inside the stent leg tip 107 and the fabric covering 113 surrounding the tip. The coaptation stitch 134 is seen to pass through the hole 121 in the stent leg tip and is approximately centered above the tip of the leg. The points of maximum curvature 135 and 137 are seen to be in the tissue at a point just inside of the centermost extremity of the cloth covering 113. The thickness of the tissue is designated as T. The ratio of $R_c/T$ should be greater than or equal to five for extended durability.

Figure 10 is another sectional view of a place of curvature of the tissue around the stent taken along section line 10—10 in Figure 2. Again, $R_c$ indicates the radius of curvature of the tissue 21 over the top of the cloth covering 124 surrounding the base ring 104 of the stent. T indicates the thickness of the tissue and, for extended durability, the ratio $R_c/T$ should be greater than or equal to five.

The reason for the above stated criteria of tip narrowness is that most curvature of the tissue around the stent leg occurs at the tips 107, 109 and 111 of the stent legs where the coaptation stitches 134, 130 and 132 in Figure 1 pull the cusps together. Thus, stress in the tissue is concentrated where the radius of curvature is smallest as can be visualized in examining Figures 7 and 4. Because the tissue formed around the legs and inside of the coaptation stitches is mobile, a large radius through which the tissue can flex helps to reduce the risk of fatigue failures.

Referring to Figure 11, there is shown another embodiment of a coaptation stitch arrangement typical for all three stent legs. This embodiment includes a separate coaptation stitch 134 passing through the tissue leaflets 50 and 70, through the top hole 121a in the tab 120, and up and over the tip 107 of the stent leg 106. Coaptation stitch 134 could be a group of stitches. Another coaptation stitch 135, or group of stitches, passes through the tissue leaflets 50 and 70, through the bottom hole 121b in the tab 120, and out and around the outside edge 138 of the stent leg 106. Thus, the top coaptation stitch 134 lies in a plane parallel to the long axis of the stent leg 106. The top stitch 134 or group of stitches is tied off above the tip 107 of the stent leg. The bottom coaptation stitch 135 lies in a plane generally perpendicular to the plane of the top stitch or stitches 134 and is tied off at the outside edge 138 of the stent leg 106. These stitches are placed so that they do not interfere with the normal operation of the valve in the open position.

Because the thread is smaller in diameter than the holes 121, the thread of coaptation stitches 134 and 135 will pull to the side of the holes 121 closest to the stitch knot when the thread is pulled tight. At times during manufacture, the thread may be passed through the tissue leaflets along an axis not parallel to the axis of the bottom hole 121b. Thus, when the thread is pulled tight the tissue leaflet on one side of the hole is moved farther toward the knot at the outside edge 138 of the stent leg 106 than is the tissue leaflet on the other side. Thus uneven pulling can cause wrinkling of the tissue leaflets.

An improvement of the coaptation stitching of Figure 11 is illustrated in Figure 12 which shows the preferred embodiment of the coaptation stitching arrangement. Figure 12 shows a coaptation stitch or stitches 140 residing generally in a plane parallel to the long axis of the stent leg 106 and passing through both the top and bottom holes 121a and 121b and through the tissue leaflets 50 and 70. The stitch can be either a single figure eight stitch as shown in Figure 12 or it can be two separate stitches, each passing through both tissue leaflets and through one of the holes 121a or 121b. Each stitch 140 is tied together above the tip 107 of the stent leg 106. The figure eight stitch shown in Figure 12 is the preferred embodiment, however, because it is simpler and faster to implement than two separate stitches both in a vertical plane. The figure eight stitch is simpler because only one knot need be tied.

The coaptation stitch illustrated in Figure 12 tends to eliminate the tendency for variations in stitch placement during manufacturing which can cause wrinkling of the tissue leaflets.

Referring again to Figure 12, it has been found experimentally that the width, $W_T$, of the tab and the amount of inward projection or protuberance, P, of the tab from the inside edge of the hole toward the center of the flow aperture of the valve is important in preventing abrasion of the tissue leaflets on the inside edges of the tab. When the tissue leaflets coapt together during the closing action of the valve, if the tabs 120, 123 and 125 protrude too far in toward the center of the flow aperture, abrasion can occur in the area generally marked 142 in Figure 8. To prevent this abrasion, the dimension P shown in Figures 5 and 12 is, in the preferred embodiment, restricted to a maximum of forty thousandths of an inch (0.040 inches or 1.016 millimeters). However, any embodiment will be satisfactory wherein the distance which the tab extends toward the center of the aperture is restricted to a distance which substantially eliminates touching between the tissue leaflets and the bottom surfaces or lower 20% of the tab under maximum backpressure conditions. The bottom of the tab surfaces refers generally to those portions of the tab surfaces below the midway point in the height of the tab designated $H_{T2}$ in Figures 6, 13 and 14.

The area of coaptation of the tissue leaflets designated generally as 144 in Figure 8, tends to grow larger during higher backpressure conditions. This phenomenon can be visualized in placing the fingertips on each hand together fingerprint to fingerprint with the fingertips on the other hand to form a roof shaped arrangement. The fingerprint area of contact represents the area of coaptation 144 in Figure 8. As the hands are pressed together keeping the fingers stiff, the fingers tend to flex toward each other such that the opposing first and second knuckle areas tend to come closer together. This represents the situation when higher backpressure exists on the tissue leaflets during closing.

As the tissue leaflets come closer together under increased backpressure, the area of contact between the leaflets tends to increase by expanding in the downward direction, i.e. toward the stent ring 104 in Figure 7. If the tabs 120, 123 and 125 extend too far in toward the center, abrasion between the mobile areas of the tissue leaflets just inside the tabs and the bottom portions of the tabs can occur. Restriction of the distance which the tabs protrude into the flow aperture tends to eliminate the aforestated abrasion.

The same reasoning applies to restriction of the relative widths, $W_T$, of the tabs 120, 123 and 125 throughout their height $H_{T2}$ as compared to the relative width of the stent legs 106, 108 and 110 as the stent legs descend from tips 107, 109 and 111. Referring to Figure 13, there is shown a detail view of the preferred embodiment for the tabs and stent leg tips. As seen in Figure 13, the width, $W_T$, of the tab 120 remains constant throughout its height, $H_{T2}$, regardless of the width of the stent leg 106. It is seen in Figure 13 that the width of the stent leg 106 is increasing at points farther away from the tip 107.

Figure 14 shows another embodiment for the tabs wherein the width, $W_T$, of the tab 120 decreases at points farther down from the tip 107 irrespective of the width of said stent leg.

The purpose of maintaining a constant or decreasing width for the tabs 120, 123 and 125 is to minimize the possibility of abrasion of the tissue leaflets on the tabs during closure of the valve and coaptation of the tissue leaflets just inside the tabs. The increasing width of the stent legs 106, 108 and 110 tend to give shape and support to the tissue leaflets to form the cusps of the valve. The increasing width of the stent legs versus the constant or decreasing width of the tabs tends to keep the tissue leaflets away from the lower surfaces of the tabs during coaptation thereby minimizing abrasion. Any shape or form for the tabs which accomplishes the purpose of minimizing or eliminating this abrasion will be satisfactory and is intended to be included within the scope of the claims appended thereto.

As exemplary only and not in any limiting sense, optimum stent dimensions for the stent depicted in Figures 5 and 6 are given in Table I. In Figures 5 and 6, D refers to the inside diameter of the stent ring and $D_o$ refers to the outside diameter thereof. U refers to the depth of scallop 112 and W refers to the width of stent ring 104. $R_i$ refers to the inside radius of the scallop 112 and $R_o$ refers to the outside radius of the scallop forming the bottom of stent ring 104. Finally $H_{T2}$ refers to the total height of the tabs.

It will be apparent that the foregoing description, given in considerable detail as to the method of carrying out the best mode of the invention as contemplated by the inventor, is given to exemplify the concepts and principles of the invention and not to limit it. The stent may be made of titanium, Delrin, polyacetal, polypropylene or Elgiloy with the fabric covering of Dacron or Teflon but the invention is not limited to these materials nor is it limited to any particular covered stent. Delrin, Elgiloy, Dacron and Teflon are trademarks. Similarly, the structures and elements of the invention have been described, in their best mode embodiments, as integral, in the case of the stent, and separate, in the case of the leaflets.

TABLE I

Stent Dimensions
(mm)
(Page 1 of 2)

| Nominal Valve Size | Outside Diameter $D_o$ (Fig. 5) | Inside Diameter D (Fig. 5) | Height H (Fig. 6) | Ring Thickness $T_R$ (Fig. 5) | Ring Width W (Fig. 6) |
|---|---|---|---|---|---|
| 15 | 13.51 | 11.99 | 10.41 | 0.762 | 2.67 |
| 17 | 15.49 | 13.97 | 11.43 | 0.762 | 2.67 |
| 19 | 17.53 | 16.00 | 12.45 | 0.762 | 2.92 |
| 21 | 19.56 | 17.78 | 13.46 | 0.889 | 2.92 |
| 23 | 21.336 | 19.56 | 14.48 | 0.889 | 2.92 |
| 25 | 23.37 | 21.34 | 15.75 | 1.016 | 3.18 |
| 27 | 25.40 | 23.36 | 16.76 | 1.016 | 3.18 |
| 29 | 27.30 | 25.02 | 18.03 | 1.143 | 3.43 |
| 31 | 29.51 | 27.23 | 19.25 | 1.143 | 3.43 |
| 33 | 31.24 | 28.96 | 20.62 | 1.143 | 3.68 |

TABLE I (cont.)
Stent Dimensions
(mm)
(Page 2 of 2)

| Scallop Depth U (Fig. 6) | Inside Radius $R_i$ (Fig. 6) | Outside Radius $R_o$ (Fig. 6) | Leg Tip Width $W_T$ (Fig. 5) | U/D | Tab Thickness $T_T$ (Fig. 6) | Tab Height HT1 (Fig. 6) | HT2 |
|---|---|---|---|---|---|---|---|
| 7.74 | 5.13 | 7.42 | 0.761 | 0.646 | 1.65 | 2.79 | 3.56 |
| 8.76 | 6.07 | 8.36 | 0.761 | 0.627 | 1.65 | 2.79 | 3.56 |
| 9.53 | 6.93 | 9.47 | 0.761 | 0.596 | 1.65 | 2.79 | 3.56 |
| 10.54 | 7.72 | 10.26 | 0.889 | 0.593 | 1.78 | 3.20 | 4.06 |
| 11.56 | 8.28 | 10.82 | 0.889 | 0.591 | 1.78 | 3.63 | 4.57 |
| 12.57 | 9.53 | 12.32 | 1.016 | 0.589 | 1.78 | 4.04 | 5.08 |
| 13.59 | 10.29 | 13.08 | 1.016 | 0.582 | 1.78 | 4.44 | 5.59 |
| 14.61 | 11.20 | 14.25 | 1.016 | 0.584 | 1.90 | 4.85 | 6.10 |
| 15.82 | 12.19 | 15.24 | 1.14 | 0.581 | 1.90 | 5.28 | 6.60 |
| 16.94 | 13.21 | 16.51 | 1.14 | 0.585 | 2.03 | 5.69 | 7.11 |

## Claims

1. A prosthetic heart valve (10) comprising: a stent (102) comprising an annular base (104) integrally formed with three upwardly extending legs (106, 108, 110), the space between the legs configured to form generally elliptically shaped scallops (112) having a depth U measured from the top of the legs (107, 109, 111) to the bottom of the scallop (112), the stent (102) circumferentially forming a substantially right cylinder having an interior diameter D with the legs (106, 108, 110) extending parallel to the axis of the cylinder, the lower edge of the base (104) forming scallops corresponding generally to the arc of the scallops (112) between the legs (106, 108, 110), the scallops of the lower edge of the base (104) and the scallops (112) between the legs vertically defining three generally elliptically shaped one-third portions of the base (104) between the respective upright legs (106, 108, 110); a fabric covering (120, 122, 124, 126) encompassing and following the configuration of the stent (102); an annular sewing ring (200) attached to the fabric covering (126) and extending outwardly from the base (104); a tissue valve element (20) formed of three tissue leaflets (30, 50, 70) joined to form a generally cylindrical three-cusp valving element (20), with said cusps (30, 50, 70) being in face-to-face contact in the closed position of the valve (10) along radial lines (133) from the centre of the valve (10) to said legs (106, 108, 110); means (210) attaching the tissue valve element (20) around the fabric-covered stent (102); and a plurality of coaptation stitches (130, 132, 134) through the tissue leaflets (30, 50, 70) adjacent the upper edges (40, 60, 80) thereof; characterised in that said coaptation stitches (130, 132, 134) are disposed directly above the tops of the fabric-covered stent legs (106, 108, 110) fixing the leaflets (30, 50, 70) to form three cusps, the tissue valve element (20) forms in the open position of the valve (10) a cylinder having an inside diameter at the top of the valve (10) substantially equal to the inside diameter of the fabric-covered stent (102), and each of said leaflets (30, 50, 70) has a top edge (40, 60, 80) forming a raised truncated triangular form with a central plateau (46) and two sides (42, 44) diverging from each side of the plateau (46) to corners at their junctures with the sides (34, 36) of the leaflet (30), with said plateaus (46) extending upwardly midway between the legs (106, 108, 110) in the open position of the valve (10) and the upper edges (40, 60, 80) of the cusps (30, 50, 70) meeting in the closed position of the valve (10) with the plateaus (46) adjacent each other.

2. The prosthetic heart valve (10) of Claim 1, further characterised in that the ratio U/D is from 0.50 to 0.65.

3. The prosthetic heart valve (10) of Claim 1, further characterised in that the sewing ring (200) extends outwardly circumferentially forming an annulus vertically following the scalloped curvature of the base (104) of the stent (102).

4. The prosthetic heart valve (10) of Claim 1, further characterised in that said stent (102) is dimensioned such that in the normal operation of said valve (10) the quotient of the radius of curvature of mobile tissue in said tissue leaflets (30, 50, 70) divided by the thickness of said mobile tissue is greater than or equal to five at all points

12

of contact between said mobile tissue and said stent (102).

5. The prosthetic heart valve (10) of Claim 1, further characterised in that the width of the area of contact (90) of the cusps (30, 50, 70) in the closed position of the valve (10) is up to 2 mm. in the center of the valve and from 3 mm. to 7 mm. halfway between the center of the valve (10) and the legs of the stent (106, 108, 110).

**Patentansprüche**

1. Prothetisches Herzventil (10) verfügend über: einen Vorhang (102) mit einer kreisförmigen Basis (104) einstückig ausgebildet mit drei aufwärts gerichteten Beinen (106, 108, 110), wobei der Raum zwischen den zur Bildung im allgemeinen elliptisch gestalteter Ausbauchungen (112) konfigurierten Beinen ein tiefes U gemessen von der Oberseite (107, 109, 111) der Beine zu dem unteren Teil der Ausbauchung (112) aufweist, der Vorhang (102) umfangsseitig einen im wesentlichen aufrechten Zylinder mit einem Innendurchmesser D bildet, wobei sich die Beine (106, 108, 110) parallel zur Achse des Zylinders erstrecken, der untere Rand der Basis (104) Ausbauchungen entsprechend im allgemeinenn dem Bogen der Ausbauchungen (112) zwischen den Beinen (106, 108, 110) bildet, die Ausbauchungen des unteren Randes der Basis (104) und dis Ausbauchungen (112) zwischen den Beinen vertikal drei im allgemeinen elliptisch gestaltee 1/3-Teile der Basis (104) zwischen den zugehörigen aufrechten Beinen (106, 108, 110) begrenzen; über eine Gewebeabdeckung (120, 122, 124, 126), die die Konfiguration des Vorhangs (102) umgreift und dieser folgt; über einen kreisförmigen Nähring (200), der an der Gewebeabdeckung (126) befestigt ist und sich von der Basis (104) aus nach außen erstreckt; über ein Gewebeventilelement (20), das aus drei Gewebeblättchen (30, 50, 70) gebildet ist, die miteinander verbunden sind zur Bildung eines im allgemeinen zylindrischen Drei - Spitzen - Ventilelements (20), wobei die genannten Spitzen (35, 50, 70) in der Schließstellung des Ventils (10) in gegenseitig flächiger Berührung miteinander entlang radialer Linien (133) von Zentrum des Ventils (10) zu den genannten Beinen (106, 108, 110) stehen; über Mittel (210), die das Gewebeventilelement (20) rund um den mit Gewebe abgedeckten Vorhang (102) festhalten; und über ein Vielzahl von Koaptationsstichen (130, 132, 134) durch die Gewebeblättchen (30, 50, 70) in der Nähe der oberen Ränder (40, 60, 80) derselben, dadurch gekennzeichnet, daß die Koaptationsstiche (130, 132, 134) direkt über den oberen Enden der Gewebe abgedeckten Vorhangbeine (106, 108, 110) angeordnet sind, die die Blättchen (30, 50, 70) festlegen zur Bildung von drei Spitzen, daß das Gewebeventilelement (20) in der Öffnungsstellung des Ventils (10) einen Zylinder mit einem Innendurchmesser an der Oberseite des Ventils (10) im wesentlichen gleich dem Innendurchmesser des Gewebe bedeckten Vorhangs (102) bildet und daß jedes der genannten Blättchen (30, 50, 70) eine obere Kante (40, 60, 80) aufweist, die eine erhobene, abgestumpfte Dreiecksform mit einem zentralen Plateau (46) und zwei Seiten (42, 44) bildet, die von jeder Seite des Plateaus (46) aus zu den Ecken an ihren Verbindungen mit den Seiten (34, 36) des Blättchens (30) divergieren, wobei sich die genannten Plateaus (46) nach oben mitten zwischen den Beinen (106, 108, 110) in der Öffnungsstellung des Ventil (10) erstrecken und sich die oberen Ränder (40, 60, 80) der Spitzen (30, 50, 70) in der Schließstellung des Ventils (10) mit den Plateaus (46) einander benachbart treffen.

2. Prothetisches Herzventil (10) nach Anspruch 1, dadurch gekennzeichnet, daß das Verhältnis U/D im Bereich von 0,50 bis 0.65 liegt.

3. Prothetisches Herzventil (10) nach Anspruch 1, dadurch gekennzeichnet, daß sich der Nähring (200) nach außen erstreckt und umfangsseitig einen Kreiring bildet, der rechtwinklig der ausgebauchten Wölbung der Basis (104) des Vorhangs (102) folgt.

4. Prothetisches Herzventil (10) nach Anspruch 1, dadurch gekennzeichnet, daß der genannte Vorhang (102) solchermaßen bemessen ist, daß im normalen Betrieb des Ventils (10) der Quotient des Wölbungsradius des bewegbaren Gewebes in dem genannten Gewebeblättchen (30, 50, 70) dividiert durch die Dicke des genannten bewegbaren Gewebes größer oder gleich 5 ist an allen Berührungspunkten zwischen dem bewegbaren Gewebe und dem genannten Vorhang (102).

5. Prothetisches Herzventil (10) nach Anspruch 1, dadurch gekennzeichnet, daß die Weite des Berührungsbereichs (90) der Spitzen (30, 50, 70) in der Schließstellung des Ventil (10) bis zu 2 mm in Zentrum des Ventils und von 3 bis 7 mm auf dem halben Weg zwischen dem Zentrum des Ventils (10) und den Beinen des Vorhangs (106, 108, 110) mißt.

**Revendications**

1. Valve cardiaque prothétique (10) comprenant: un cadre (102) comportant une base annulaire (104) réalisée d'une seule pièce avec trois pieds (106, 108, 110) s'étendant vers le haut, l'espace entre les pieds étant configuré pour former des festons (112) de forme globalement elliptique ayant une profondeur U mesurée du sommet des pieds (107, 109, 111) au fond du feston (112), le cadre (102) formant circonférentiellement un cylindre sensiblement droit ayant un diamètre intérieur D, les pieds (106, 108, 110) s'étendant parallèlement à l'axe du cylindre, le bord inférieur de la base (104) formant des festons correspondant globalement à l'arc des festons (112) entre les pieds (106, 108, 110), les festons du bord inférieur de la base (104) et les festons (112) entre les pieds définissant verticalement trois tiers, de forme globalement elliptique, de la base (104) entre les pieds respectifs (106, 108, 110) orientés vers le haut; un revêtement d'étoffe (120, 122, 124, 126) entourant et suivant la configuration du cadre (102); une bague annulaire (200) à coudre attachée au revêtement d'étoffe (126) et s'étendant vers l'extérieure de la base (104); un élément (20) de valve tissulaire formé de trois lamelles (30, 50, 70) de tissue reliées pour former un élément valvulaire tricuspide (20) globalement cylindrique, lesdites cuspides (30, 50, 70) étant en contact face à face dans la position fermée de la valve (10) suivant des lignes radiales (133) allant du centre de la valve (10) auxdits pieds (106, 108, 110); des moyens (210) attachant l'élément de valve tissulaire (20) autour du cadre (102) revêtu d'étoffe; et plusieurs points de coaptation (130, 132, 134) traversant les lamelles tissulaires (30, 50, 70) à proximité immédiate de leurs bords supérieurs (40, 60, 80); caractérisée en ce que lesdits points de coaptation (130, 132, 134) sont disposés directement au-dessus des sommets des pieds (106, 108, 110) du cadre recouvert d'étoffe, fixant les lamelles (30, 50, 70) pour former trois cuspides, l'élément de valve tissulaire (20) forme dans la position ouverte de la valve (10) un cylindre ayant un diamètre intérieur, au sommet de la valve (10), sensiblement égal au diamètre intérieur du cadre (102) revêtu d'étoffe, et chacune desdites lamelles (30, 50, 70) présente un bord supérieur (40, 60, 80) constituant une forme triangulaire tronquée surélevée avec un plateau central (46) et deux côtés (42, 44) divergeant de chaque côté du plateau (46) jusqu'à des angles situés à leur jonction avec les côtés (34, 36) de la lamelle (30), lesdits plateaux (46) s'étendant vers le haut à midistance entre les pieds (106, 108, 110) dans la position ouverte de la valve (10) et les bords supérieurs (40, 60, 80) des cuspides (30, 50, 70) se rejoignant dans la position fermée de la valve (10), les plateaux (46) étant adjacents entre eux.

2. Valve cardiaque prothétique (10) selon la revendication 1, caractérisée en outre en ce que le rapport U/D est de 0,50 à 0,65.

3. Valve cardiaque prothétique (10) selon la revendication 1, caractérisée en outre en ce que la bague à coudre (200) s'étend vers l'extérieur circonférentiellement, formant un anneau suivant verticalement la courbure festonnée de la base (104) du cadre (102).

4. Valve cardiaque prothétique (10) selon la revendication 1, caractérisée en outre en ce que ledit cadre (102) est dimensionné de manière que, dans le fonctionnement normal de ladite valve (10), le quotient du rayon de courbure du tissu mobile dans leadites lamelles tissulaires (30, 50, 70) par l'épaisseur dudit tissu mobile soit supérieur ou égal à 5 en tous les points de contact entre ledit tissu mobile et ledit cadre (102).

5. Valve cardiaque prothétique (10) selon la revendication 1, caractérisée en outre en ce que la largeur de la zone de contact (90) des cuspides (30, 50, 70) dans la position fermée de la valve (10) s'élève jusqu'à 2 mm au centre de la valve et de 3 mm à 7 mm à mi-distance entre le centre de la valve (10) et les pieds du cadre (106, 108, 110).

Fig. 1

Fig. 2

CENTER OF STENT

Fig. 4

Fig. 10

0 051 451

Fig. 5

OUTFLOW

Fig. 3

INFLOW

Fig. 6

2

**0 051 451**

Fig. 7.

Fig. 8

Fig. 9

*Fig. 12*

*Fig. 11*

*Fig. 13*

*Fig. 14*

Fig.15
(PRIOR ART)

Fig.16

Fig.17

Fig.18